# DEMANDE DE BREVET EUROPEEN

(11) **EP 0 538 535 A1**
(43) Date de publication de la demande: **28.04.1993**
(21) Numéro de dépôt: 91440111.2
(22) Date de dépôt: 27.12.1991
(51) Int. Cl.: A61F 13/15

(54) **Couche-culotte à haut pouvoir absorbant**

(30) Priorité: 16.10.1991 FR 9113155
(71) Demandeur: PROTECO SARL, F-59494 Petite Forêt (FR)
(72) Inventeur: Brutin, Jean-Marc, F-59178 Hasnon, (Nord) (FR)
(74) Mandataire: Hennion, Jean-Claude

(57) **Abrégé**

Couche-culotte (1) à haut pouvoir absorbant destinée notamment pour enfants en bas âges et pour personnes incontinentes.

Selon l'invention, le tampon (4) comporte des moyens comprenant au moins une matière absorbante (21-23) et au moins une matière superabsorbante (22-24) pour assurer un gradient de diffusion et d'absorption notamment aux liquides depuis la feuille perméable (3) jusqu'à la feuille imperméable (2) afin de rendre la feuille interne perméable (2) moins imprégnée des dits liquides.

## Description

La présente invention a pour objet une couche-culotte à fort pouvoir absorbant qui trouvera son application dans le domaine de l'hygiène infantile ou de l'incontinence.

Une couche-culotte doit être suffisamment absorbante pour éviter que la peau de l'enfant ou de la personne qui la porte ne soit humide. Elle doit aussi être suffisamment confortable pour éviter à l'utilisateur d'être gêné dans ses mouvements. Par ailleurs, elle doit rester correctement positionnée sur le corps de l'utilisateur pendant ses mouvements.

Pour cela, on a réalisé des couches-culottes qui comportent d'une façon générale les éléments suivants:
- une feuille externe imperméable notamment aux liquides en polyéthylène ou en polypropylène,
- une feuille interne perméable au contact de la peau en fibres non tissées,
- inséré entre les feuilles un tampon absorbant réalisé en un matériau tel que de la fibre de cellulose.

La capacité d'absorption des liquides, qui est l'une des caractéristiques essentielles des couches-culottes, est actuellement obtenue en utilisant un tampon absorbant qui présente une épaisseur suffisante et en renforçant la tenue de la couche au niveau de la ceinture et du passage des jambes notamment par des élastiques.

Pour accroître les performances des couches-culottes et notamment leur capacité d'absorption, une première solution consiste à augmenter la masse de la matière absorbante généralement en fibre de cellulose.

Cette technique dans laquelle la nappe de matière absorbante est surdimensionnée présente un inconvénient qui est celui d'une épaisseur importante.

Cette surépaisseur de la couche a pour conséquence de la rendre plus rigide et moins confortable pour la personne qui la porte.

Par ailleurs, au moment de leur commercialisation, ces couches-culottes plus volumineuses doivent être conditionnées dans des emballages spécifiques ou bien en nombre plus limité dans des emballages classiques. C'est la raison pour laquelle cette technique a très vite été abandonnée.

Une autre technique plus satisfaisante consiste à utiliser une masse absorbante qui présente par elle-même un fort pouvoir d'absorption et de rétention.

Pour cela, on associe à la masse absorbante un additif constitué par des micro-grains ou des micro-fibres plus communément connus sous le nom de matière superabsorbante.

Il s'agit généralement de polyacrylate de sodium ou de potassium obtenu par des techniques de polymérisation classiques.

Ces composés présentent une capacité d'absorption de liquides importante qui peut être égale à plusieurs dizaines de fois leur poids.

Toutefois, de tels additifs sont onéreux. De plus, ils sont très abrasifs, ce qui a pour conséquence d'entraîner une usure importante des appareils de coupe lors de la réalisation des couches sur la chaîne de fabrication.

En effet, la réalisation de couches-culottes selon des cadences importantes en continu nécessite de multiples opérations de coupe réalisées généralement au moyen de couteaux précis et onéreux.

Pour diminuer le coût de ces couches-culottes, on a pensé à rendre absorbantes ces couches uniquement dans des zones localisées correspondant généralement à la morphologie de la personne en fonction de son sexe. Pour se faire, on dispose préférentiellement la matière superabsorbante dans de telles zones localisées à savoir sensiblement dans la partie médiane des entrejambes pour des couches-culottes destinées à des filles et sensiblement à l'extrémité des entrejambes pour des couches-culottes destinées à des garçons..

Toutefois, la pratique a montré que l'utilisation de ces matières superabsorbantes ne procurait pas tous les résultats escomptés.

En effet, l'augmentation de la concentration de telles matières ne peut dépasser un seuil très faible au-delà duquel on constate un phénomène de blocage de la diffusion et de l'absorption.

Ce phénomène résulte de la transformation de la poudre de polyacrylate de sodium ou de potassium lorsqu'elle est saturée en un gel qui forme un écran imperméable au passage des liquides.

On constate ainsi, la présence en surface d'une micro-couche de matière superabsorbante saturée. Cette couche superficielle de matière superabsorbante saturée s'oppose à la diffusion du liquide en profondeur de sorte que la majeure partie de la matière superabsorbante disposée sous cette couche ne peut jouer son rôle.

Il en résulte que la diffusion des liquides au sein de l'ensemble de la masse absorbante vers la feuille imperméable ne peut se faire dans de bonnes conditions. Il se produit alors une saturation de la masse absorbante en surface en contact de la feuille perméable ce qui a pour conséquence de former une zone humide au contact de la peau de l'utilisateur.

L'utilisation de matières superabsorbantes telles que des polyacrylates de sodium ou de potassium pour améliorer les performances de la couche-culotte n'apporte donc pas entière satisfaction.

Le but de la présente invention est de proposer une couche-culotte à haut pouvoir absorbant qui permette de pallier aux inconvénients des techniques connues actuellement.

C'est ainsi qu'elle permet un gradient d'absorption des liquides depuis la feuille perméable au contact de la peau de l'utilisateur jusqu'à la feuille externe imperméable qui élimine la présence d'humidité au niveau de la feuille externe afin de rendre la peau de la personne qui porte la couche plus sèche.

Un autre but de la présente invention est de fournir une couche-culotte d'un confort accru et d'une qualité améliorée dont la durée d'utilisation peut être prolongée ce qui se traduit par de meilleures performances économiques.

Un autre but de la présente invention est également de fournir un procédé de fabrication de couches-culottes conformes à l'invention qui permet de remédier à l'usure des couteaux de coupe résultant de la présence d'une matière superabsorbante.

Un autre avantage de la présente invention est de proposer un procédé de fabrication rapide à partir d'une chaîne de fabrication classique sans modification des machines existantes c'est-à-dire sans augmentation du coût de production.

Un autre avantage de la présente invention est également de proposer un procédé de fabrication rapide d'un coussin absorbant à partir d'un matériel compatible avec la chaîne de fabrication existante qui permette de réaliser des couches-culottes destinées à des personnes de sexes différents.

Selon l'invention, la couche-culotte à haut pouvoir absorbant destinée notamment pour enfants en bas âges et pour personnes incontinentes du type comprenant une feuille externe imperméable, une feuille interne perméable, et un tampon absorbant inséré entre la feuille externe et la feuille interne, est caractérisée en ce que le tampon comporte des moyens comprenant au moins une matière absorbante et au moins une matière superabsorbante pour assurer un gradient de diffusion et d'absorption notamment aux liquides depuis la feuille perméable jusqu'à la feuille imperméable afin de rendre la feuille interne perméable moins imprégnée des dits liquides.

L'invention a également pour objet un procédé de réalisation d'une telle couche-culotte, caractérisée en ce que pour préparer le coussin tridimensionnel,
- on divise finement une matière absorbante,
- on mélange intimement une matière superabsorbante avec la matière absorbante finement divisée,
- on réalise une empreinte tridimensionnelle du mélange,
et en ce que pour réaliser l'ensemble du tampon absorbant :
- on pulvérise une matière superabsorbante sur la surface d'une nappe d'une matière absorbante sensiblement dans sa partie médiane,
- on rapporte le coussin tridimensionnel,
- on assujettit l'ensemble de la nappe de matière absorbante et le coussin tridimensionnel rapporté.

D'autres buts et avantages de la présente invention apparaîtront au cours de la description qui va suivre qui n'est cependant donnée qu'à titre indicatif et qui n'a pas pour but de la limiter.

L'invention sera bien comprise à la lecture de la description suivante accompagnée de dessins en annexe parmi lesquels :
- la figure 1 est une vue schématique illustrant une couche-culotte en position ouverte,
- la figure 2 est une vue en coupe selon la ligne II-II de la figure 1,
- la figure 3 est une vue schématique illustrant un coussin conforme à l'invention,
- les figures 4 à 6 sont des vues schématiques illustrant les étapes de réalisation du coussin,
- les figures 7 à 10 sont des vues schématiques illustrant les étapes de réalisation de l'ensemble du tampon absorbant.

La présente invention vise une couche-culotte à haut pouvoir absorbant qui trouvera son application dans le domaine de l'hygiène infantile et médicale.

Bien que l'invention ait été développée dans le cadre de réalisation de couches-culottes, elle trouvera néanmoins son application dans des produits similaires tels que des changes complets ou autres.

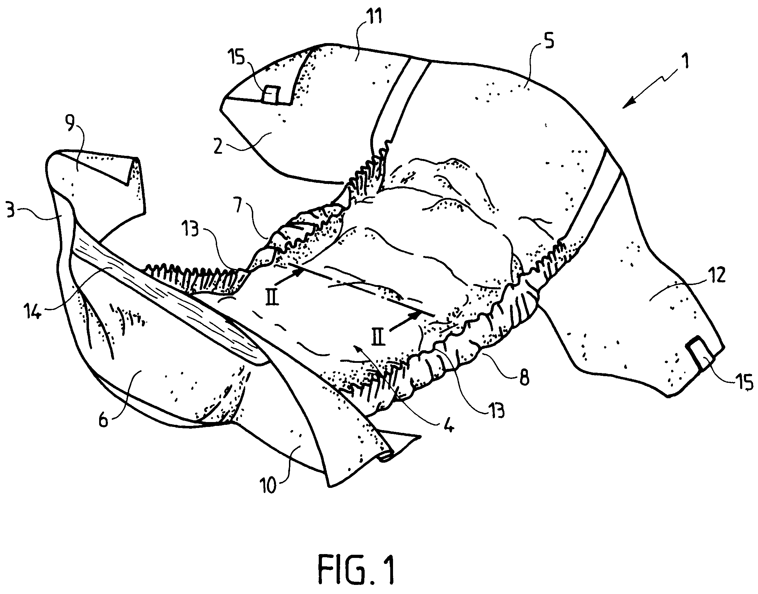

En se référant à la figure 1, on voit une couche-culotte 1 à jeter destinée notamment pour les enfants en bas âges ou pour les personnes incontinentes.

Cette couche-culotte comprend :
- une feuille externe 2 imperméable aux liquides,
- une feuille interne 3 perméable qui vient en contact de la peau de la personne qui porte la couche,
- un tampon absorbant 4 fixé entre la feuille externe et la feuille interne 3.

La feuille interne 3 et la feuille externe 2 ainsi que le tampon absorbant 4 sont réalisés en des matériaux couramment utilisés dans le domaine des couches-culottes.

Par exemple, la feuille interne 3 peut être réalisée en toute sorte de matières tendres, flexibles, souples et poreuses, laissant en outre passer les fluides. Elles peuvent comprendre un voile de fibres non tissées, un papier en soie résistant à l'humidité et une feuille de filaments tissés, etc.... Cette feuille peut subir des traitements de surface afin d'accroître ses capacités de transfert des fluides notamment par des agents tensio-actifs ou par tous autres agents similaires.

La feuille externe 2 est imperméable notamment aux fluides. Elle peut comprendre un voile ou une feuille fine de film en matière plastique comme par exemple polyéthylène, polypropylène, polychlorure de vinyle ou en tout autre agent équivalent. Elle peut être transparente ou présenter un aspect de surface mate ou gaufrée.

Le tampon absorbant 4 peut être réalisé en une matière absorbante telle que notamment en un matériau cellulosique ou dans le cas d'un tampon aéroformé en fibres de bois.

La feuille externe 2 est fixée sur les parties latérales et externes de la feuille interne 4 par des lignes ou des points de colle longitudinaux. Elle peut également l'être par d'autres techniques telles que par des lignes ou des points de soudure à ultrasons ou par thermoscellage. De même, on peut envisager de solidariser la feuille externe directement à la face externe du matelas absorbant par toute technique connue et appropriée.

La couche-culotte 1 présente une forme générale de I ou de sablier. Elle comporte une partie avant 5 et une partie arrière 6 séparée par une découpe 7, 8 latérale permettant le passage des jambes de la personne lorsque la couche est en position d'utilisation.

La partie avant 5 et la partie arrière 6 présentent chacune deux oreilles 9, 10, 11, 12 symétriques disposées de part et d'autre d'un plan médian vertical.

Des éléments élastiques latéraux 13 sont posés longitudinalement de manière linéaire de chaque côté des bords de matelas absorbant 4 au niveau des découpes 7, 8.

Dans un mode de réalisation, ces éléments élastiques peuvent être constitués par des brins d'une gomme caoutchoutée telle du latex. Ils sont assujettis sur toute leur longueur à la face interne de la feuille imperméable 2 par des adhésifs tels que de la colle.

Les extrémités des brins 13 sont libres et ne se rétractent qu'au moment de l'opération finale lors de leur découpe transversale correspondant à la découpe de la couche-culotte. L'obtention de ces résultats est obtenue par des techniques classiques. On imagine que, dans des variantes de réalisation, on pourrait utiliser tout autre type d'éléments élastiques qui présentent un nombre de brins déterminés ou qui se présentent sous forme également de bandes.

La couche-culotte 1 comporte également une bande transversale de ceinture élastique 14 qui est disposée dans la partie avant et collée sur toute la largeur de cette partie sur sa face externe de la feuille imperméable 2. Cette fixation est réalisée par des points de colle mais elle peut être également réalisée par toute autre technique appropriée telle que par des points ou des lignes de soudure à ultrasons ou bien encore par thermoscellage.

Une languette adhésive 15 est assujettie par des points de colle dans la partie supérieure de la face externe de la feuille imperméable 2 sur chacune des oreilles 11 et 12.

Ces languettes 15 sont destinées à venir adhérer à la bande de ceinture 14 pour permettre la mise en position de la couche dans une position d'utilisation.

En effet, la couche-culotte peut alors au moment de l'utilisation facilement être ajustée grâce à une traction convenable sur les bandes adhésives 15 qui transmettent l'effort directement à la bande de ceinture 14 élastique disposée sur la partie avant de la couche.

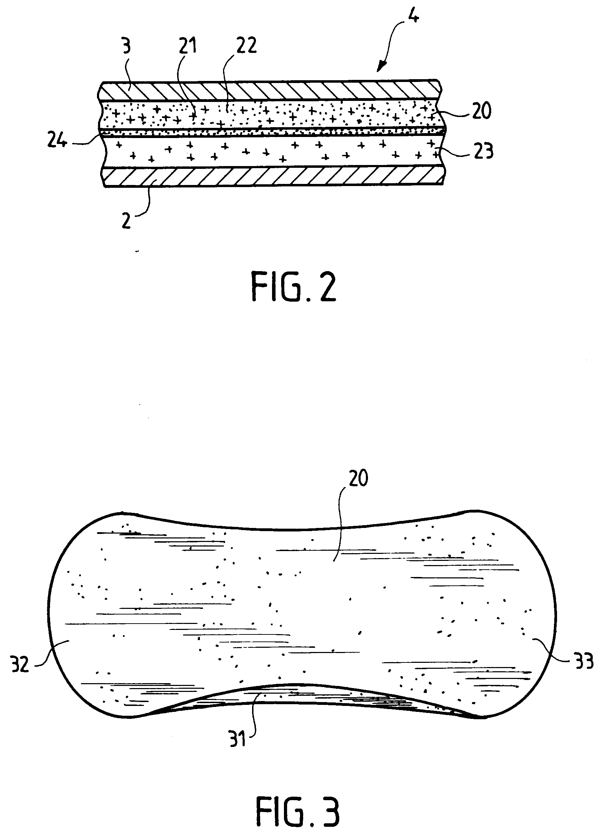

Selon l'invention, comme plus particulièrement illustrée à la figure 2, le tampon absorbant 4 comporte des moyens pour assurer un gradient de diffusion et d'absorption des liquides depuis la feuille perméable 3 jusqu'à la feuille imperméable 2 afin de rendre la feuille interne perméable 3 moins imprégnée des dits liquides.

Les moyens pour assurer ce gradient de diffusion et d'absorption sont constitués par un coussin tridimensionnel 20 rapporté sur la feuille interne 2 qui comporte un mélange intime d'une matière absorbante finement divisée telle que par exemple des fibres de cellulose 21 et une matière superabsorbante pulvérulente 22 et par une nappe 23 disposée vers la feuille externe 3 de la matière absorbante, nappe 23 qui est recouverte sensiblement dans sa partie médiane d'une répartition superficielle d'une matière superabsorbante pulvérulente 24.

La matière superabsorbante 22-24 peut être par exemple un polymère hydrophile à base d'acide acrylique et d'acrylate de métal alcalin. Il peut s'agir par exemple d'un polymère hydrophile en micro-perles à base d'acide acrylique et d'acrylate de métal alcalin constitués par un copolymère d'acide acrylate de potassium ou de calcium réticulé avec un ou plusieurs réticulants.

On sait que le comportement du polyacrylate de sodium ou de potassium en présence d'humidité pose certains problèmes étant donné qu'il se gélifie. Notamment si sa densité au sein du matériau absorbant 4 est importante, la présence d'une quantité importante de liquides transforme cet additif pulvérulent en une couche de gel plus ou moins étanche. Cette couche de gel s'oppose alors au passage ultérieur des liquides et ainsi toute une partie du matériau absorbant devient inutilisée.

La présente invention remédie à cet inconvénient en utilisant des moyens d'absorption et de diffusion qui assurent un gradient d'absorption et des liquides au sein de la masse absorbante du tampon 4 conforme à l'invention.

Dans un premier mode de réalisation, la substance superabsorbante 24 qui est répartie superficiellement sur la nappe 23 de matière absorbante est identique à la matière superabsorbante 22 mélangée intimement avec la matière absorbante finement divisée 21 constituant le coussin 20.

Dans une variante de réalisation, la matière superabsorbante 24 répartie superficiellement sur la nappe 23 de matière absorbante est différente de la matière superabsorbante 22 mélangée intimement avec la matière absorbante 21 finement divisée constituant le coussin 20. Toutefois, quel que soit le mode de réalisation utilisé, le tampon absorbant 4 conforme à l'invention évite la formation de gel superficiel et permet d'assurer une diffusion des liquides afin de rendre la feuille interne perméable 3 moins imprégnée des dits liquides de sorte que la peau de la personne qui porte la couche soit toujours au sec.

En effet, en présence du liquide, il y a tout d'abord diffusion rapide au travers du coussin tridimensionnel 20 sans formation d'un gel bloquant puis, diffusion beaucoup plus lente et absorption du liquide au sein de la nappe de matière absorbante 23 en contact avec la feuille externe 2 imperméable. Cette diffusion et cette absorption selon un gradient du liquide permettent ainsi de rendre la peau de la personne qui porte la couche sèche.

Ce gradient de diffusion et d'absorption est rendu possible grâce à la structure différente du coussin et de la nappe de matière absorbante constituant le tampon absorbant 4. A cet effet, le coussin absorbant tridimensionnel rapport 20 est constitué par un mélange intime d'une matière absorbante 22 finement divisée et d'un additif superabsorbant 21 de telle sorte que cela lui confère une structure homogène dans laquelle le superabsorbant 21 est disposé aléatoirement dans l'ensemble de l'épaisseur de ce coussin tridimensionnel 20.

Par ailleurs, la structure de la nappe disposée vers la feuille externe 2 est constituée par une matière absorbante recouverte sensiblement dans sa partie médiane d'une répartition superficielle d'une matière superabsorbante pulvérulente 24. Cette répartition superficielle s'entend d'une répartition en surface ou en quasi-surface selon une dispersion homogène ou irrégulière des granulés ou de la poudre.

Ainsi, selon l'invention, la combinaison du coussin tridimensionnel rapporté 20 qui surmonte la nappe 23 permet d'assurer l'absorption et la diffusion selon un gradient sélectif des liquides de sorte que les caractéristiques d'absorption de la couche-culotte sont améliorées.

Naturellement, dans l'esprit de la présente invention, il est possible d'utiliser un tampon absorbant 4 qui présente un nombre plus important de nappes de matière absorbante de même qu'éventuellement un nombre supérieur de coussin tridimensionnel rapporté 20. Toutefois, l'épaisseur de ce tampon absorbant 4 doit resté compatible avec les caractéristiques de confort escomptées pour de tels produits.

C'est la raison pour laquelle selon l'invention, le tampon absorbant comporte uniquement un seul coussin tridimensionnel 20 rapporté ainsi qu'une unique nappe 23 de matière absorbante recouverte superficiellement d'une matière superabsorbante 24.

Le coussin tridimensionnel rapporté 20 qui est plus particulièrement représenté à la figure 3, présente une forme sensiblement d'un sablier dont la partie médiane 31 présente une épaisseur supérieure à celle de ses extrémités 32, 33.

A ce sujet, dans d'autres modes de réalisation, il est possible d'adapter cette forme du coussin tridimensionnel 20 en fonction de la conformation et de la configuration propres de la couche-culotte 1.

Pour accroître encore les performances de la couche-culotte 1 conforme à l'invention, on dispose ce coussin 20 dans une partie appropriée de la partie située entre les échancrures 7 et 8 latérales définissant les entrejambes.

C'est ainsi que pour des personnes ou des enfants de sexe masculin, on dispose le coussin tridimensionnel 20 sensiblement au niveau des extrémités de la partie délimitée par les échancrures 7, 8 latérales permettant le passage des jambes. De même, pour des personnes ou des enfants de sexe féminin, on place préférentiellement ce coussin rapporté tridimensionnel 20 sensiblement dans la partie médiane des échancrures 7, 8 latérales permettant le passage des jambes.

L'invention a également pour objet un procédé de réalisation de couches-culottes. Dans ce procédé, pour réaliser un coussin tridimensionnel :
- on divise finement une matière absorbante,
- on mélange intimement une matière superabsorbante avec la matière absorbante finement divisée,
- on réalise une empreinte tridimensionnelle du mélange sous forme d'un coussin
et pour réaliser l'ensemble du tampon absorbant :
- on pulvérise une matière superabsorbante sur la surface d'une nappe d'une matière absorbante sensiblement dans sa partie médiane,
- on rapporte le coussin tridimensionnel,
- on assujettit ensemble la nappe de matière absorbante et le coussin tridimensionnel.

En se reportant maintenant aux figures 5 à 7, on va décrire un mode de réalisation du coussin tridimensionnel 20 rapporté.

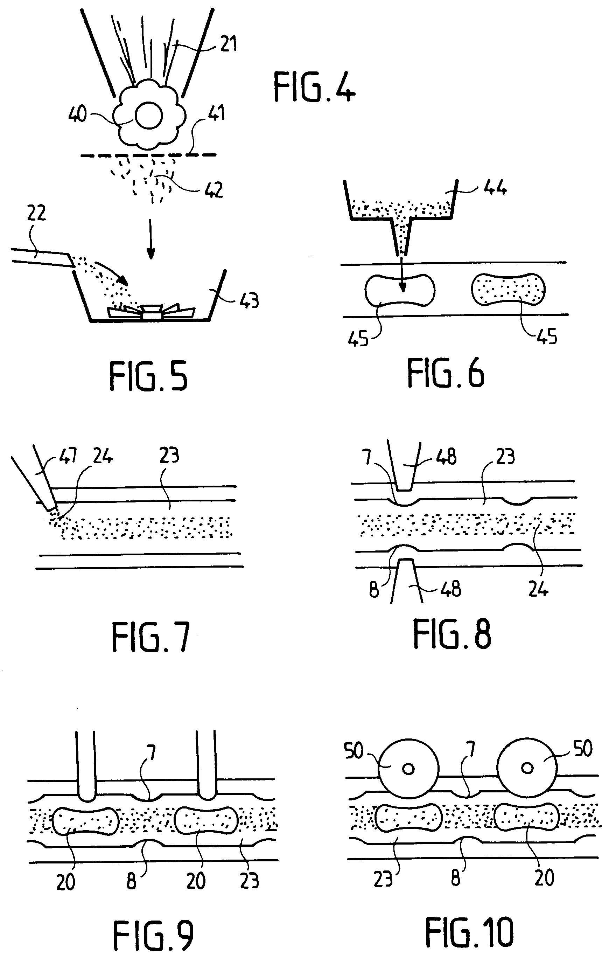

Dans ce mode de réalisation comme illustré à la figure 4, on divise finement une matière absorbante 21 telle que de la pâte à papier défibrée ou par toute autre matière absorbante. Cette matière absorbante 21 est convoyée sous la forme d'une bande généralement jusqu'au niveau d'un broyeur 40 associé à un tamis 41 dont les grilles permettent d'obtenir une granulométrie des fibres 42 de matières absorbantes 21 divisées souhaitée.

On mélange comme montrée à la figure 5 ces fibres 42 de matière absorbante 21 finement divisées avec une matière superabsorbante 22. Ce mélange s'effectue à l'intérieur d'un mélangeur 43 d'un type classique. Bien entendu, le temps de mélange ainsi que la vitesse de rotation des moyens de malaxage sont ajustables afin d'obtenir un mélange homogène 44 des fibres 42 de matières absorbantes 21 finement divisées et du superabsorbant 22.

On réalise ensuite comme cela est représenté à la figure 6 à partir de ce mélange une empreinte 45.

Pour ce faire, le mélange homogène 44 de matière absorbante divisé 21 et du superabsorbant 22 est convoyé depuis le mélangeur 43 jusque dans des empreintes 45 à l'intérieur desquelles ce produit est placé.

Cette réalisation du coussin tridimensionnel 20 peut être réalisée en continu ou de manière séquentielle.

En se référant maintenant aux figures 7 à 10, on va décrire la réalisation du tampon absorbant 4 proprement dit.

On fait défiler en continu, comme illustrée à la figure 7, une bande 23 de matière absorbante telle que de la pâte à papier défibrée, et on pulvérise au moyen d'un canon pulvérisateur 47, d'une buse ou par tout autre moyen équivalent une matière superabsorbante 24 sensiblement sur la surface de la nappe 23 de matière absorbante. Afin de rendre plus économique le procédé, ainsi que de limiter l'usure ultérieure des couteaux lors de la découpe des entrejambes, cette pulvérisation est focalisée sensiblement dans la partie médiane de la nappe 23 de matière absorbant

On découpe ensuite les entrejambes 7, 8 comme cela est illustré à la figure 8.

Cette découpe s'effectue au moyen d'outils classiques tels que des couteaux 48 à des intervalles réguliers et en continu.

Grâce à la focalisation et à la localisation de la matière superabsorbante 24 sur la nappe 23 de matière absorbante, on découpe les entrejambes 7, 8 dans une zone qui ne présente pas de matière superabsorbante 24 de sorte que l'usure des couteaux 48 est limitée ce qui permet de réduire le coût de fabrication ainsi que d'accroître les performances. En effet, dans les techniques classiques, en raison du caractère abrasif de la matière superabsorbante disposée sur la nappe de matière absorbante, les couteaux devaient être remplacés souvent ce qui avait pour conséquence d'immobiliser la chaîne de fabrication.

Puis, on rapporte le coussin tridimensionnel 20 comme illustré à la figure 9. Pour ce faire, les coussins tridimensionnels 20 réalisés comme décrits précédemment sont convoyés par des techniques classiques et disposés dans la partie adaptée en fonction de la destination ultérieure des couches.

Pour ce faire, on place préférentiellement le coussin tridimensionnel 20 sensiblement dans la partie médiane de la couche-culotte lorsque celle-ci est destinée à des personnes de sexe féminin. De même l'on dispose préférentiellement le coussin tridimensionnel 20 sensiblement aux extrémités de la couche-culotte lorsque celle-ci est destinée à des personnes de sexe masculin.

Enfin, on solidarise la nappe de matière absorbante 23 et le coussin 20 tridimensionnel rapporté comme cela est illustré à la figure 10. Cette solidarisation se fait au moyen d'un calandrage par des rouleaux 50.

Bien entendu d'autres techniques équivalentes pourraient être mises en oeuvre et notamment on peut assujettir le coussin tridimensionnel 20 et la nappe 23 de matière absorbante par d'autres techniques telles que par des soudures à ultrasons ou par collage.

Dans un mode de réalisation, afin de limiter encore plus le coût de fabrication de ces couches, on récupère la matière absorbante résultant de la découpe des entre-jambes pour la diviser et pour qu'elle entre dans la réalisation des coussins tridimensionnels 20.

En outre, il faut noter que la fabrication du tampon 4 est réalisée de préférence en continu. Elle peut être faite en même temps que celle du coussin tridimensionnel 20 ou bien indépendamment de la réalisation de ce coussin tridimensionnel 20.

D'autres mises en oeuvre de la présente invnetion, à la portée de l'homme de l'art, auraient pu être également envisagées sans pour autant sortir du cadre de celle-ci.

## Revendications

1. Couche-culotte (1) à haut pouvoir absorbant destinée notamment pour enfants en bas âges et pour personnes incontinentes du type comprenant une feuille externe imperméable (2), une feuille interne perméable (3) et un tampon absorbant (4) inséré entre la feuille externe (2) et la feuille interne (3), caractérisée en ce que le tampon (4) comporte des moyens comprenant au moins une matière absorbante (21-23) et au moins une matière superabsorbante (22-24) pour assurer un gradient de diffusion et d'absorption notamment aux liquides depuis la feuille perméable (3) jusqu'à la feuille imperméable (2) afin de rendre la feuille interne perméable moins imprégnée des dits liquides.

2. Couche-culotte selon la revendication 1, caractérisée en ce que les moyens pour assurer un gradient de diffusion et d'absorption sont constitués par au moins un coussin tridimensionnel (20) rapporté vers la feuille interne (3) qui comporte un mélange intime d'une matière absorbante (21) finement divisée et d'une matière superabsorbante (22) pulvérulente et par au moins une nappe (23) de matière absorbante disposée vers la feuille externe (2) et recouverte sensiblement dans sa partie médiane d'une répartition superficielle d'une matière superabsorbante (24) pulvérulente.

3. Couche-culotte selon la revendication 1, caractérisée en ce que le coussin (20) rapporté a une forme tridimensionnelle sensiblement de sablier dont la partie médiane (31) présente une épaisseur supérieure à celle de ses extrémités (32-33).

4. Couche-culotte selon la revendication 1, caractérisée en ce que le superabsorbant (24) réparti superficiellement sur la nappe de matière absorbant (23) est identique au superabsorbant (22) mélangé intimement avec la matière absorbante (21) finement divisée constituant le coussin.

5. Couche-culotte selon la revendication 1, caractérisée en ce que le superabsorbant (24) réparti superficiellement sur la nappe de matière absorbant (23) est différent du superabsorbant (22) mélangé intimement avec la matière absorbante (21) finement divisée constituant le coussin.

6. Couche-culotte selon la revendication 1, caractérisée en ce qu'elle comporte un coussin (20) rapporté disposé sensiblement dans sa partie médiane des entrejambes (7-8).

7. Procédé de réalisation de couches-culottes selon la revendication 1, caractérisé en ce que pour réaliser le coussin (20) tridimensionnel :
- on divise finement une matière absorbante (21),
- on mélange intimement une matière superabsorbante (22) avec la matière absorbante (21) finement divisée,
- on réalise une empreinte tridimensionnelle du mélange sous forme d'un coussin (20)
et que pour réaliser l'ensemble du tampon absorbant :
- on pulvérise une matière superabsorbante (24) sur la surface d'une nappe (23) d'une matière absorbante sensiblement dans sa partie médiane,
- on rapporte le coussin tridimensionnel (20),
- on assujettit l'ensemble de la nappe de matière absorbante et le coussin tridimensionnel (20) rapporté.

8. Procédé selon la revendication 7, caractérisé en ce qu'on découpe dans une zone sans matière superabsorbante (24) de la nappe (23) de matière absorbante des éléments pour confectionner des entrejambes,

9. Procédé selon la revendication 9, caractérisé en ce que l'on récupère les découpes d'entrejambes pour les diviser finement en vue de réaliser le coussin tridimensionnel (20).

10. Procédé selon la revendication 7, caractérisé en ce que l'on rapporte le coussin tridimensionnel (20) localement en fonction du sexe de l'utilisateur de la couche.
